# EUROPEAN PATENT APPLICATION

(11) **EP 4 455 270 A1**
(43) Date of publication of application: **30.10.2024**
(21) Application number: 22912033.2
(22) Date of filing: 23.12.2022
(51) Int. Cl.: C12N 5/071, A61K 35/36, A61P 17/14, A61K 8/98, A61Q 7/00

(54) **METHOD FOR CULTURING HAIR FOLLICLE-DERIVED STEM CELLS, AND USE THEREOF**

(30) Priority: 24.12.2021 KR 20210187682
(71) Applicant: Ewha University-Industry Collaboration Foundation, Seoul 03760 (KR); Hierabio Inc., Seoul 03760 (KR)
(72) Inventor: LEE, Seung Jin, Seoul 07333 (KR); SHIN, Ji-young, Gyeryong-si, Chungcheongnam-do 32800 (KR); JEON, Jeong Hwa, Seoul 03938 (KR); KIM, Heejung, Seongnam-si, Gyeonggi-do 13503 (KR)
(74) Representative: Studio Torta S.p.A.
(86) International application number: PCT/KR2022/021214
(87) International publication number: WO 2023/121400

(57) **Abstract**

The present invention relates to a method for culturing hair follicle-derived stem cells and uses thereof, particularly to a method for culturing hair follicle-derived stem cells; hair follicle-derived stem cells prepared by the method; and a pharmaceutical composition for hair loss treatment or hair growth promotion; a quasi-drug composition; and a cosmetic composition, which contains the prepared hair follicle-derived stem cells, a culture of the hair follicle-derived stem cells, or cells differentiated from the hair follicle-derived stem cells as an active ingredient.

According to the culture method of the present invention, stem cells can be effectively isolated and obtained from hair follicle tissue, and the obtained hair follicle-derived stem cells have excellent pluripotency and self-replication ability as well as excellent hair loss treatment and hair growth promotion effects and can be usefully utilized for hair loss improvement, prevention, or treatment.

## Description

### [Technical Field]

The present invention relates to a method for culturing hair follicle-derived stem cells and uses thereof, particularly to a method for culturing hair follicle-derived stem cells; hair follicle-derived stem cells prepared by the method; and a pharmaceutical composition for hair loss treatment or hair growth promotion; a quasi-drug composition; and a cosmetic composition, which contains the prepared hair follicle-derived stem cells, a culture of the hair follicle-derived stem cells, or cells differentiated from the hair follicle-derived stem cells as an active ingredient.

### [Background Art]

Stem cells are cells in a stage before differentiating into the respective cells that make up a tissue, and are cells that can proliferate indefinitely in an undifferentiated state and have the potential to differentiate into cells of various tissues by specific differentiation stimulation.

Stem cells are largely divided into embryonic stem cells (ES cells) and adult stem cells (tissue-specific stem cells) depending on the differentiation potential. Embryonic stem cells are stem cells isolated from the inner cell mass (ICM), which will develop into a fetus, of a blastocyst embryo, the initial stage in which the fertilized egg is formed but is not yet implanted in the endometrium, and are cells that have the potential to differentiate into cells of all tissues.

Meanwhile, tissue-specific stem cells are stem cells specific to each organ that appear in the stage in which the embryonic development process progresses and each organ of the embryo is formed, and their differentiation potency is generally limited (multipotent) only to the cells that make up the tissue. Representative tissue specific stem cells include hematopoietic stem cells, which exist in bone marrow, and mesenchymal stem cells, which differentiate into connective tissue cells other than blood cells. Hematopoietic stem cells differentiate into various blood cells such as red blood cells and white blood cells, and mesenchymal stem cells differentiate into osteoblasts, chondroblasts, adipocytes, and myoblasts.

Recently, since the success of the isolation of embryonic stem cells from humans, interest in their clinical application has increased. The application of stem cells that is receiving the most attention is the use of stem cells as a cell source for cell replacement therapy.

### [Disclosure]

### [Technical Problem]

The existing hair follicle stem cell isolation method is an enzymatic isolation method using collagenase and the like (Korean Patent Publication No. 10-2014-0075469 A), which has the disadvantage of affecting cell performance since the damage to proteins on the cell surface is inevitable, and it is thus required to study skin stem cell isolating and culturing methods.

Against this background, the present inventors have conducted various studies to develop new stem cells, as a result, found out that hair follicle-derived stem cells prepared by the culture method of the present invention have excellent in vitro proliferation ability, cell performance, and differentiation potency, and can be used for hair loss treatment and hair growth promotion, and thus completed the present invention.

### [Technical Solution]

An object of the present invention is to provide a method for culturing hair follicle-derived stem cells using hair follicle tissue and a hydrogel.

Another object of the present invention is to provide hair follicle-derived stem cells prepared by the method.

Still another object of the present invention is to provide a pharmaceutical composition for hair loss treatment or hair growth promotion, containing the hair follicle-derived stem cells, a culture of the hair follicle-derived stem cells, or cells differentiated from the hair follicle-derived stem cells as an active ingredient.

Still another object of the present invention is to provide a quasi-drug composition for hair loss improvement or hair growth promotion, containing the hair follicle-derived stem cells, a culture of the hair follicle-derived stem cells, or cells differentiated from the hair follicle-derived stem cells as an active ingredient.

Still another object of the present invention is to provide a cosmetic composition for hair loss improvement or hair growth promotion, containing the hair follicle-derived stem cells, a culture of the hair follicle-derived stem cells, or cells differentiated from the hair follicle-derived stem cells as an active ingredient.

Still another object of the present invention is to provide a composition for hair follicle-derived stem cell culture, containing hair follicle tissue and a hydrogel.

Still another object of the present invention is to provide a kit for hair follicle-derived stem cell culture, containing the composition for stem cell culture.

### [Advantageous Effects]

According to the culture method of the present invention, stem cells can be effectively isolated and obtained from hair follicle tissue, and the obtained hair follicle-derived stem cells have excellent pluripotency and self-replication ability as well as excellent hair loss treatment and hair growth promotion effects and can be usefully utilized for hair loss improvement, prevention, or treatment.

### [Brief Description of Drawings]

FIG. 1 illustrates the results (FIG. 1A) of fragment formation of hair follicle tissue, a schematic diagram (FIG. 1B) of encapsulation and culture of hair follicle tissue in a hydrogel, and the results (FIG. 1C) of cell migration and proliferation in a hydrogel by culture period;
FIG. 2 illustrates the results of examining the immunophenotype of HB-hair follicle-derived stem cells;
FIG. 3 illustrates the results of examining the proliferation ability of HB-hair follicle-derived stem cells when subculture is performed;
FIG. 4 illustrates the results of examining the colony formation ability of HB-hair follicle-derived stem cells;
FIG. 5A illustrates the results of examining the differentiation potency of HB-hair follicle-derived stem cells into adipocytes;
FIG. 5B illustrates the results of examining the differentiation potency of HB-hair follicle-derived stem cells into osteocytes;
FIG. 6A illustrates the results of DB-based hair growth function-related gene String Network analysis and the results of identifying specific hair growth promotion functional genes of HB-hair follicle-derived stem cells;
FIG. 6B illustrates the comparative analysis results of the expression levels of major hair growth function-related genes in HB-hair follicle-derived stem cells;
FIG. 6C illustrates the comparative analysis results of the expression levels of hair growth promotion functional genes specifically expressed in HB-hair follicle-derived stem cells to those in adipose-derived stem cells;
FIG. 6D illustrates the comparative analysis results of the expression levels of hair growth promotion functional genes specifically expressed in HB-hair follicle-derived stem cells to those in adipose-derived stem cells;
FIG. 6E illustrates the comparative analysis results of the expression levels of hair growth promotion functional genes specifically expressed in HB-hair follicle-derived stem cells to those in existing hair follicle-derived stem cells;
FIG. 7A illustrates the results of examining the hair growth promotion effect of HB-hair follicle-derived stem cells in a mouse model;
FIG. 7B illustrates the results of examining the hair growth promotion effect of HB-hair follicle-derived stem cells in a mouse model;
FIG. 8A illustrates the results of examining the hair follicle formation ability of HB-hair follicle-derived stem cells in a mouse model;
FIG. 8B illustrates the results of examining the hair follicle formation ability of HB-hair follicle-derived stem cells in a mouse model;
FIG. 8C illustrates the results of examining the hair follicle formation ability of HB-hair follicle-derived stem cells in a mouse model;
FIG. 8D illustrates the results of examining the hair follicle formation ability of HB-hair follicle-derived stem cells in a mouse model;
FIG. 9A illustrates the results of examining the Wnt signaling mechanism of HB-hair follicle-derived stem cells in a mouse model; and
FIG. 9B illustrates the results of examining the Wnt signaling mechanism of HB-hair follicle-derived stem cells in a mouse model.

### [Detailed Description of Preferred Embodiments]

Hereinafter, the present invention will be described in detail. Meanwhile, each description and each embodiment disclosed in the present invention may also be applied to other descriptions and other embodiments, respectively. That is, all combinations of various elements disclosed in the present invention fall within the scope of the present invention. Further, the scope of the present invention is not limited by the following specific description.

An aspect of the present invention that aims to achieve the objects provides a method for culturing hair follicle-derived stem cells using a hair follicle tissue and a hydrogel, and hair follicle-derived stem cells cultured by the method.

Specifically, the method for culturing hair follicle tissue-derived stem cells includes (a) encapsulating hair follicle tissue in a hydrogel and culturing the hair follicle tissue to obtain a culture; and (b) decomposing the hydrogel in the obtained culture to recover stem cells that have migrated from the hair follicle tissue into the hydrogel and proliferated in the hydrogel.

In the present invention, the hydrogel refers to a three-dimensional network structure formed by crosslinking hydrophilic polymers through covalent or non-covalent bonds. As hair follicle tissue is three-dimensionally encapsulated in a hydrogel, the hydrogel may provide physical support to the hair follicle tissue, and at the same time, provide an extracellular matrix function that allows hair follicle-derived stem cells present in the hair follicle tissues to migrate into and proliferate in the hydrogel.

The hydrogel of the present invention is preferably a phase transitional hydrogel that exists in a solution state and can be converted into sol and gel for hydrogel-supported three-dimensional culture, and may specifically be one or more selected from the group consisting of collagen, gelatin, chondroitin, hyaluronic acid, alginic acid, Matrigel^{™}, chitosan, peptide, fibrin, PGA (polyglycolic acid), PLA (polylactic acid), PEG (polyethylene glycol), and polyacrylamide, but is not limited thereto.

In step (a), the culture of a hydrogel in which hair follicle tissue is encapsulated may be performed after the hydrogel in which hair follicle tissue is encapsulated is immersed in a conventional medium known to be suitable for stem cell culture in the art.

Step (a) may involve encapsulating hair follicle tissue in a multilayered hydrogel and culturing the hair follicle tissue. Step (a) may specifically involve encapsulating hair follicle tissue in a double-layered hydrogel and culturing the hair follicle tissue, but is not limited thereto.

In Example of the present invention, when hair follicle tissue was encapsulated in a hydrogel, the hydrogel was added in two stages so that the hair follicle tissue was encapsulated in the sandwich-type double-layered hydrogel, and culture was performed.

In the case of encapsulating hair follicle tissue in a multilayered hydrogel as described above, there are advantages that it is possible to prevent the tissue fragment from not being hydrated but floating due to the hydrophobicity of the outer layer of the hair cuticle remaining in the hair follicle tissue fragment and to sufficiently encapsulate the hair follicle tissue in the double-layered hydrogel by the two-stage hydrogel treatment.

In step (a), a fragment obtained by removing the site corresponding to the follicular infundibulum from the epidermis may be used as the hair follicle tissue. The fragment may be 0.01 to 10 mm, 0.05 to 5 mm, or 0.1 to 3 mm, but the size of the fragment is not limited.

In Example of the present invention, when a hair follicle tissue fragment was formed, a 1 to 3 mm hair follicle tissue fragment obtained by removing the site corresponding to the infundibulum of the epidermal region was used for culture.

In step (a), the culture period of hair follicle tissue may be 3 to 20 days, 3 to 18 days, 3 to 16 days, 3 to 14 days, or 4 to 12 days, but is not limited thereto.

As the culture period increases, the number of isolated cells per tissue weight increases, which is advantageous in terms of cell isolation efficiency. However, as the culture continues, cell confluency in the hydrogel surrounding the tissue increases and this leads to the possibility of inhibiting cell proliferation and inducing stem cell differentiation by contact inhibition as well as the cell/hydrogel ratio increases, cell aggregation occurs, and cell release is inhibited by selective decomposition of hydrogel during cell isolation.

Hence, it is important to appropriately adjust the culture period to a period during which cell outgrowth is observed when a tissue is cultured since aggregation due to cell overcrowding around the tissue and turbidity around the tissue are observed.

In Example of the present invention, hair follicle tissue was cultured for 4 to 12 days and used.

For hair follicle tissue culture in step (a), the hair follicle tissue may be seeded with an inter-tissue spacing of 1 mm or more, 2 mm or more, 3 mm or more, 4 mm or more, 5 mm or more, 50 mm or less, 40 mm or less, 30 mm or less, 20 mm or less, or 10 mm or less, but is not limited thereto.

When the tissue is seeded for culture, the inter-tissue spacing is required to be adjusted in consideration of the outgrown distance and the distance of cells that have migrated/proliferated from one tissue.

In Example of the present invention, tissue fragments were seeded with an inter-tissue spacing of 5 mm or more and cultured.

In step (b), only the hydrogel may be selectively decomposed in order to recover hair follicle-derived stem cells that have migrated/proliferated in the hydrogel after the hydrogel-supported three-dimensional culture step of step (a).

The hydrogel may be decomposed through one or more enzymes selected from the group consisting of collagenase, gelatinase, urokinase, streptokinase, TPA (tissue plasminogen activator), plasmin, and hyaluronidase.

The hair follicle stem cell isolation/culture technology of the present invention is a hydrogel-based niche preservation self-replication induced isolation technology, which isolates stem cells, and corresponds to a new technology that allows hair follicle tissue to survive/culture in vitro and to isolate high-purity cells directly from the tissue without enzyme treatment.

Unlike existing methods, this isolation method differs from existing methods and has the advantage of being superior in terms of in vitro proliferation ability and cell performance.

Cells isolated by existing methods can be amplified in vitro only up to 2 to 3 passages (maximum 5 passages), but cells isolated by the method of the present invention have excellent performance that cellular senescence does not occur when the cells are subcultured 8 passages or more and can be subcultured for a long period of time, and mass production of the cells is possible.

Meanwhile, hair follicle-derived stem cells prepared by the method for preparing hair follicle-derived stem cells provided by the present invention exhibit immunological characteristics where CD73, CD90, CD105, Nestin, and Lgr5 are expressed but CD34, CD45, and HLA-DR are not expressed, and exhibit pluripotent characteristics such that the hair follicle-derived stem cells can differentiate into adipocytes, osteocytes, and the like, but are not limited thereto.

The hair follicle-derived stem cells may be used interchangeably with hair follicle-derived stem cells prepared by the method for preparing hair follicle-derived stem cells provided by the present invention, hair follicle-derived stem cells of the present invention, hair follicle-derived stem cells, HB-hair follicle-derived stem cells, HB-hair follicle-derived stem cells of the present invention, and HB-HFSC.

In Example of the present invention, it was possible to verify the proliferation ability, differentiation potency, and colony formation ability of hair follicle-derived stem cells prepared by the method for preparing hair follicle-derived stem cells of the present invention.

In Example of the present invention, it was possible to verify the hair growth promotion effect of hair follicle-derived stem cells prepared by the method for preparing hair follicle-derived stem cells of the present invention.

In Example of the present invention, it was further possible to verify that hair follicle-derived stem cells prepared by the method for preparing hair follicle-derived stem cells of the present invention had a long survival period in vitro and excellent viability through the evaluation of cell growth ability/cellular senescence (in vitro proliferative power) and purity of primary cells when subculture was performed.

Hair follicle-derived stem cells prepared by the method for preparing hair follicle-derived stem cells provided by the present invention can express specific hair growth promotion functional genes.

At this time, the hair growth promotion functional genes may specifically be one or more genes selected from SANP25(Synaptosome Associated Protein 25), COL10A1 (Collagen Type X Alpha 1 Chain), TMEM119 (Transmembrane Protein 119), AQP1 (Aquaporin 1), PLCB4(1-Phosphatidylinositol-4,5-bisphosphate phosphodiesterase beta-4), or ITGA8(Integrin Subunit Alpha 8), but is not limited thereto, and may include any gene known to be a hair growth promotion functional gene.

In Example of the present invention, it was found that the expression levels of the seven hair growth promotion functional genes in hair follicle-derived stem cells prepared by the method for preparing hair follicle-derived stem cells were as remarkable as 10-fold or more compared to those in adipose-derived stem cells, and it was possible to verify that hair follicle-derived stem cells prepared by the method of the present disclosure exhibited specific hair growth promotion functional genes.

Another aspect of the present invention provides a pharmaceutical composition for hair loss treatment or hair growth promotion, containing the hair follicle-derived stem cells, a culture of the hair follicle-derived stem cells, or cells differentiated from the hair follicle-derived stem cells as an active ingredient.

In the present invention, the term "culture" or "culture solution" may be a culture obtained during or after culture of hair follicle-derived stem cells in a medium, or its supernatant, its concentrate, or its lyophilized product.

In the present invention, the term "hair growth" or "hair growth promotion" is a concept that may include not only the effects of promoting hair growth, increasing the number of hairs, and thickening hair but also hair follicle regeneration or proliferation of hair follicle cells, and the proliferation of hair follicle cells may include promoting the conversion of hair follicle cells from the telogen phase to the anagen phase, but the hair growth or hair growth promotion is not limited thereto.

In the present invention, the term "pharmaceutical composition" refers to a product prepared for the purpose of preventing or treating a disease, and may be administered in various oral and parenteral dosage forms in actual clinical administration, and may be prepared using commonly used diluents or excipients such as fillers, extenders, binders, wetting agents, disintegrants, and surfactants in the case of being formulated.

Depending on the formulation, pharmaceutically acceptable additives may be further contained, and at this time, as the pharmaceutically acceptable additives, starch, gelatinized starch, microcrystalline cellulose, lactose, povidone, colloidal silicon dioxide, calcium hydrogen phosphate, lactose, mannitol, taffy, gum arabic, pregelatinized starch, corn starch, powdered cellulose, hydroxypropyl cellulose, Opadry, sodium starch glycolate, lead carnauba, synthetic aluminum silicate, stearic acid, magnesium stearate, aluminum stearate, calcium stearate, white sugar, dextrose, sorbitol, and talc may be used. The pharmaceutically acceptable additives according to the present invention may be contained by 0.1 to 90 parts by weight based on the composition.

In addition to the hair follicle-derived mesenchymal stem cells, the pharmaceutical composition may further contain one or more conventional pharmaceutically acceptable inert carriers, for example, preservatives, analgesics, solubilizers, stabilizers or the like in the case of injections and bases, excipients, lubricants, preservatives or the like in the case of preparations for topical administration.

The pharmaceutical composition of the present invention may be administered as an individual therapeutic agent or in combination with other therapeutic agents, and may be administered sequentially or simultaneously with conventional therapeutic agents. The pharmaceutical composition of the present invention may be administered a single time or multiple times. It is important to administer the pharmaceutical composition of the present invention in a minimum amount that can afford the maximum effect as well as does not cause side effects, taking all the factors into consideration, and this minimum amount can be easily determined by a person skilled in the art.

In addition, the pharmaceutical composition provided by the present invention may further contain various ingredients that assist hair loss treatment or hair growth promotion, maintain the activity of the hair follicle-derived stem cells, or promote differentiation of the hair follicle-derived stem cells other than the hair follicle-derived stem cells, as an example, may further contain anti-inflammatory agents, stem cell mobilization factors, growth inducing factors, and the like.

The pharmaceutical composition of the present invention may be used for the purpose of improving, preventing or treating hair loss.

Hair loss of the present invention includes both non-scarring alopecia, which is temporary hair loss, and cicatricial alopecia, which occurs when hair follicles or hair roots are permanently destroyed, and non-scarring alopecia includes infectious alopecia, traumatic alopecia, inflammatory alopecia, congenital alopecia, endocrine alopecia, neoplastic alopecia, nutritional deficiency alopecia, hair loss caused by drugs, and hair loss caused by hair structural abnormalities, and also includes male pattern baldness, female pattern baldness, and alopecia areata.

In the present invention, the term "improvement" may refer to any action that delays the progression of hair loss or alleviates symptoms of hair loss by administering the composition according to the present invention to an individual.

In the present invention, the term "prevention" may refer to any action that suppresses or delays the occurrence of hair loss by administering the composition according to the present invention to an individual.

In the present invention, the term "treatment" may refer to any action that improves or benefits hair loss symptoms by administering the composition of the present invention to an individual suspected of having hair loss.

Still another object of the present invention is to provide a method for hair growth promotion or hair loss prevention or treatment, which includes administering to an individual a composition containing the hair follicle-derived stem cells, a culture of the hair follicle-derived stem cells, or cells differentiated from the hair follicle-derived stem cells as an active ingredient.

The hair follicle-derived stem cells, culture of the hair follicle-derived stem cells, or cells differentiated from the hair follicle-derived stem cells, hair growth promotion, hair loss, prevention, and treatment are as described above.

In the present invention, the term "administration" means introducing the composition into an individual in an appropriate manner. Specifically, a composition containing the hair follicle-derived stem cells of the present invention, a culture of the hair follicle-derived stem cells, or cells differentiated from the hair follicle-derived stem cells as an active ingredient, or the pharmaceutical composition containing the same may be applied to a local site, for example, to a hair loss site or administered as an injection, but the administration is not limited thereto.

The composition may be used in combination with other pharmaceutical compositions that can be used to promote hair growth or prevent or treat hair loss.

In the present invention, the term "individual" means any animal such as rats, mice, and livestock, including humans, that has or may develop hair loss. The animal may be a human as well as a mammal, such as a cow, horse, sheep, pig, goat, camel, antelope, dog, or cat in need of prevention or treatment of similar symptoms thereto, but is not limited thereto.

The composition of the present invention can be administered in a pharmaceutically effective amount.

The term "pharmaceutically effective amount" means an amount sufficient to treat a disease with a reasonable benefit/risk ratio applicable to medical treatment, and the effective dosage level may be determined based on factors including the type and severity of the individual, age, sex, activity of the drug, sensitivity to the drug, time of administration, route of administration and rate of excretion, period of treatment, and concurrently used drugs, and other factors well known in the medical field.

The composition may be administered as an individual therapeutic agent or in combination with other therapeutic agents, and may be administered sequentially or simultaneously with conventional therapeutic agents. The composition may be administered a single time or multiple times. It is important to administer the composition in a minimum amount that can afford the maximum effect as well as does not cause side effects, taking all the factors into consideration, and this minimum amount can be easily determined by a person skilled in the art.

The composition may be administered orally or parenterally (for example, intravenously, subcutaneously, intraperitoneally, or topically) depending on the desired method, and the dosage varies depending on the patient's condition and weight, degree of disease, drug form, and route and time of administration, but may be appropriately selected by a person skilled in the art. As a specific example, the composition can generally be administered once or several times a day, but the preferred dosage may be appropriately selected by a person skilled in the art depending on the individual's condition and weight, degree of disease, drug form, and route and period of administration.

Still another object of the present invention is to provide a quasi-drug composition for hair loss improvement or hair growth promotion, containing the hair follicle-derived stem cells, a culture of the hair follicle-derived stem cells, or cells differentiated from the hair follicle-derived stem cells as an active ingredient.

In the present invention, the term "quasi-drug composition" is an article used for the purpose of treating, alleviating, healing, or preventing diseases in humans or animals, which corresponds to one of textiles, rubber products or similar articles; articles that weakly act on the human body or do not directly act on the human body and are not instruments or machines and similar articles; and preparations used for sterilization, deinsectization, and similar purposes to prevent infection, and refers to articles used for the purpose of diagnosing, treating, alleviating, healing or preventing diseases in humans or animals excluding those that are not instruments, machines or devices and articles used for the purpose of having a pharmacological effect on the structure and function of humans or animals excluding those that are not instruments, machines, or devices, and may specifically be a skin preparation for external use or a personal care product.

Still another object of the present invention is to provide a cosmetic composition for hair loss improvement or hair growth promotion, containing the hair follicle-derived stem cells, a culture of the hair follicle-derived stem cells, or cells differentiated from the hair follicle-derived stem cells as an active ingredient.

In the present invention, the term "cosmetic composition" may be prepared in any commonly prepared formulation, for example, may be formulated as a solution, emulsion, suspension, paste, cream, lotion, gel, powder, spray, surfactant-containing cleansing product, oil, soap, liquid detergent, bath salt, foundation, makeup base, essence, toner, foam, pack, softening water, sunscreen cream, or sun oil.

Still another object of the present invention is to provide a composition for hair follicle-derived stem cell culture, containing a hair follicle tissue and a hydrogel, and a kit for hair follicle-derived stem cell culture, containing the composition.

In addition, the kit for hair follicle-derived stem cell culture may include the composition for stem cell culture and various components such as solutions and devices necessary for the culture of the stem cells.

Still another object of the present invention is to provide a use of a composition containing the hair follicle-derived stem cells, a culture of the hair follicle-derived stem cells, or cells differentiated from the hair follicle-derived stem cells as an active ingredient for hair growth promotion or hair loss prevention or treatment.

### [Mode for Carrying Out the Invention]

Hereinafter, the present invention will be described in more detail with reference to Examples. However, these Examples are for illustrative purposes only, and the scope of the present invention is not limited to these Examples.

### Example 1. Three-dimensional culture of multilayered hydrogel-supported hair follicle tissue

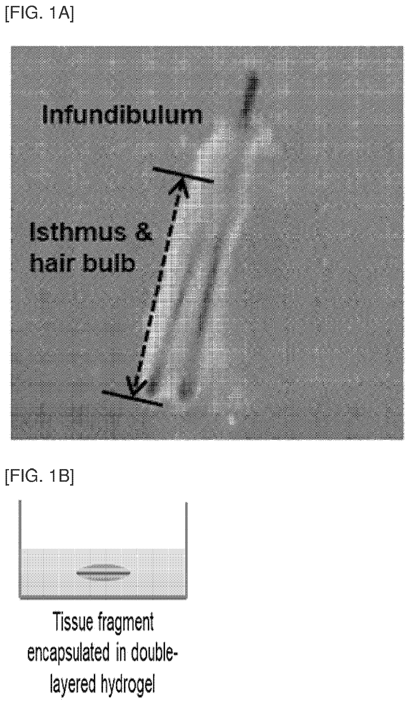

The donated hair follicle tissue, which was soaked in physiological saline solution and stored under refrigeration, was used as a sample and was washed with PBS (Gibco) three times. After the hair shaft portion of the washed hair follicle tissue was cut, 0.5 mm to 1 mm of the infundibulum portion was removed from the cut side to form a hair follicle tissue fragment. After washing with DMEM (low glucose, Gibco) once, the supernatant was removed, and 5 mL of fibrinogen solution (0.25% w/v fibrinogen (GC Biopharma) and 200 µg/mL tranexamic acid (Shin Poong Pharmaceutical) in DMEM) was added. After an equal amount of thrombin solution (0.5 IU/mL thrombin (GC Biopharma) in DMEM) was added, the mixture was immediately transferred to a 100 mm culture vessel, and the hair follicle fragment and hydrogel were spread evenly and then allowed to stand at room temperature for 10 minutes or less so that the hair follicle tissue was fixed to the hydrogel. Additionally, 5 mL of fibrinogen solution (0.25% w/v fibrinogen and 200 µg/mL tranexamic acid in DMEM) and 5 mL of thrombin solution (0.5 IU/mL thrombin in DMEM) were added. The mixture was allowed to stand for 10 minutes or less to form a secondary hydrogel, and then culture was performed at 37°C for 2 hours for sufficient gelation (FIG. 1B). A growth medium (90% low DMEM : F12 = 1 : 1, 10% Fetal bovine serum, 20 ng/mL epidermal growth factor, 5 ng/mL basic fibroblast growth factor, 10 ng/mL insulin-like growth factor, and 10 mg/mL gentamicin) containing 100 µg/mL tranexamic acid was added by 10 mL, and culture was performed for 1 hour while stirring was performed at a speed of 10 to 30 rpm. After that, the growth medium was changed once every 2 to 3 days.

### Example 2. Recovery and amplification culture of HB-hair follicle-derived stem cells migrated into and grown in multilayered hydrogel

Hair follicle tissue was cultured for 10 days or 14 days by the method described in Example 1. After the hydrogel was washed three times with warm PBS for 3 minutes, 30% FBS/DMEM was added, and stirring was performed on an orbital shaker at a speed of 10 to 30 rpm. After the supernatant was removed 1 hour later, 10 mL of 30% FBS/DMEM containing 1 µg/mL alteplase was added, and the gel was decomposed. When the gel was sufficiently decomposed, culture was performed overnight under static conditions, then the supernatant was removed, the growth medium was added, and culture was performed in a conventional manner. The growth medium was replaced every 2 to 3 days, and subculture was performed every 4 to 6 days.

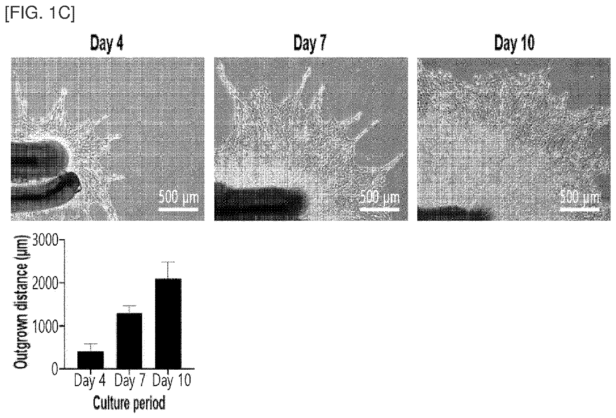

The results obtained by performing tissue culture and cell isolation and culture by the methods are illustrated in FIG. 1.

### Example 3. Examination of immunophenotype of isolated and cultured HB-hair follicle-derived stem cells

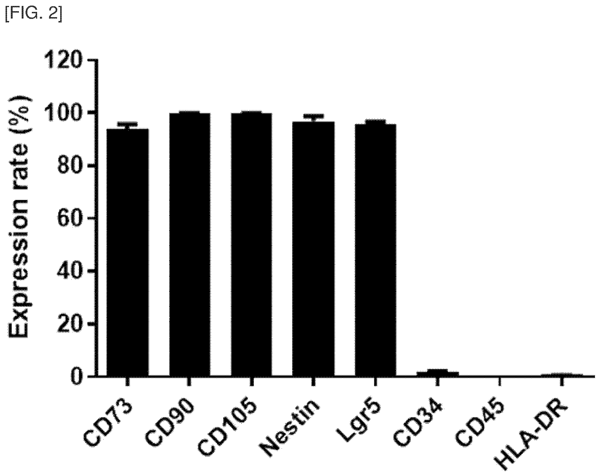

In order to examine the immunophenotype of HB-hair follicle-derived stem cells isolated and cultured by the method of Example 2, cells cultured in a T75 flask were obtained and suspended in 3% bovine serum albumin (sigma)/PBS. The cell suspension was divided into 9 equal parts and reacted with the following antibodies at 4°C for 1 hour: FITC-conjugated CD73 (Abcam, 1:25) antibody, APC-conjugated CD90 (Invitrogen, 1:40), FITC-conjugated CD105 (Abcam, 1:50), FITC-conjugated CD34 (Abcam, 1:10) antibody, FITC-conjugated HLA-DR (Novusbiologicals, 1:20) antibody, Nestin (Novusbiologicals, 1:100) antibody, FITC-conjugated Lgr5 (Origene, 1:100) antibody, FITC-conjugated mouse IgG Isotype control (Novusbiologicals, 1:5) antibody, or AlexaFluor594-conjugated CD45 (Novus, 1:20) antibody. After the reaction, the reaction mixture was centrifuged at 300 g for 5 minutes, washed, suspended in 3% BSA/PBS, and subjected to measurement using a flow cytometer (BD) (FIG. 2).

As a result, as can be seen from FIG. 2, it was found that the isolated and cultured HB-hair follicle-derived stem cells expressed CD73, CD90, CD105, Nestin, and Lgr5 but did not express CD34, CD45, and HLA-DR.

### Example 4. Examination of proliferation ability and colony formation ability of isolated and cultured HB-hair follicle-derived stem cells

In order to examine the proliferation ability of the isolated and cultured HB-hair follicle-derived stem cells obtained in Example 2, the population doubling time depending on the number of passages was measured through the number of cells obtained by seeding cells at a density of 4000 cells/cm² at the time of initial culture and subculturing the cells every 4 days.

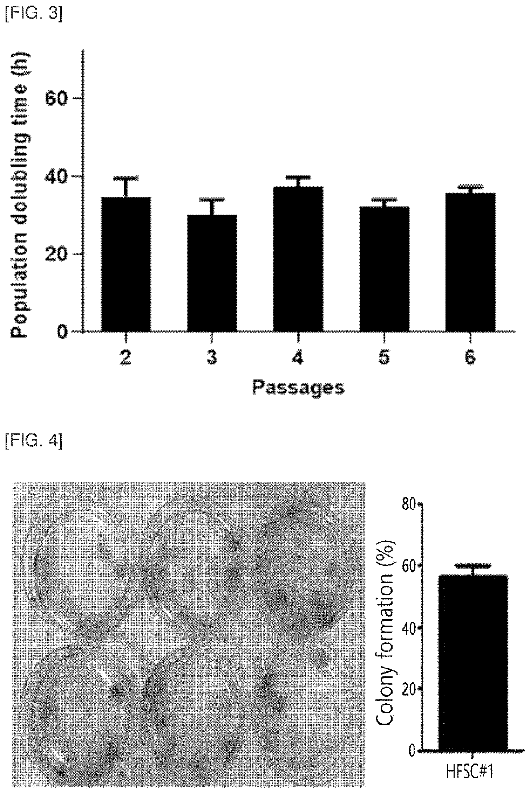

The number of cells was measured by counting the number of unstained cells by trypan blue staining, and the population doubling times (PDTs) were calculated from the following equation and illustrated in FIG. 3: PDT = [days / (logN₂ - logN₁)] / log2, (N₁ = 4,000 cells, N₂ = number of cells after 4 days).

In order to examine the colony formation ability of the isolated and cultured HB-hair follicle-derived stem cells obtained in Example 2, human hair follicle-derived stem cells (4 passages) were seeded in a 6-well plate at 20 cells/well. The cells were cultured for 14 days while the growth medium was replaced every 3 days. The cells were washed with PBS and fixed with 4% paraformaldehyde (4% PFA, Sigma) at room temperature for 15 minutes. The fixed cells were washed with PBS three times and then stained with 0.5% crystal violet/methanol at room temperature for 15 minutes. After the remaining solutions were all removed, washing with distilled water was performed three times, the stained colonies were observed, and the results are illustrated in FIG. 4.

As illustrated in FIGS. 3 and 4, it was found that the HB-hair follicle-derived stem cells obtained by the method of Example 2 maintained the proliferation ability when subculture was performed and also had the colony formation ability at a certain level or more.

In other words, when the HB-hair follicle-derived stem cells were subcultured up to the sixth passage and the population doubling time (PDT) was determined, it was found that the PDT was observed to be an average of 32 hours ± 7 hours and the proliferation ability was maintained when subculture was performed.

### Example 5. Examination of differentiation potency of HB-hair follicle-derived stem cells

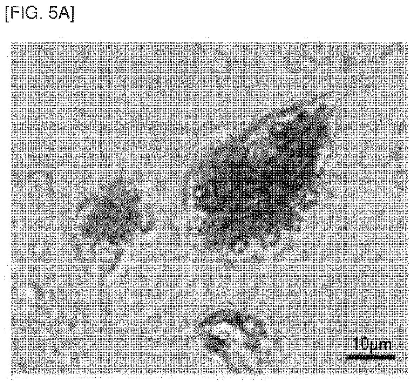

In order to evaluate the differentiation potency of the obtained HB-hair follicle-derived stem cells into adipocytes, cells were seeded at a concentration of 1 × 10⁴ cells/cm² and cultured in a growth medium until 80% confluency was achieved, then the medium was changed to StemPro^{™} Adipogenesis differentiation kit (Gibco, cat. A1007001), and culture was performed for 14 days while the medium was changed every 3 days. The cells were fixed with 4% PFA for 15 minutes and reacted with 60% isopropanol for 15 minutes, then the solutions were all removed, and the cells were thoroughly dried. The cells were stained with 60% Oil Red O (sigma)/ethanol at room temperature for 15 minutes to examine whether there was fat accumulation in the cytoplasm (FIG. 5A).

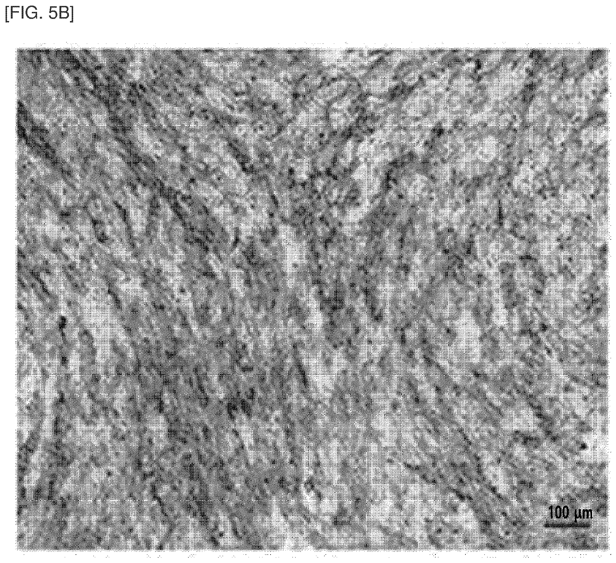

In order to evaluate the differentiation potency of the HB-hair follicle-derived stem cells into osteocytes, cells were seeded at a concentration of 1 × 10⁴ cells/cm² and cultured in a growth medium until 80% confluency was achieved, then the medium was changed to StemPro^{™} Osteogenesis differentiation kit (Gibco, cat. A1007201), and culture was performed for 14 days while the medium was changed every 3 days. The cells were fixed with 4% PFA for 60 seconds, treated with BCIP/NBT substrate solution (Sigma), and stained for 10 minutes under light-blocking conditions to examine the activity of alkaline phosphatase (ALP) (FIG. 5B).

In other words, from the results, it was verified that HB-hair follicle-derived stem cells obtained by the culture method of the present disclosure possessed pluripotency.

### Example 6. Examination of differentially expressed genes in HB-hair follicle-derived stem cells compared to adipose-derived stem cells

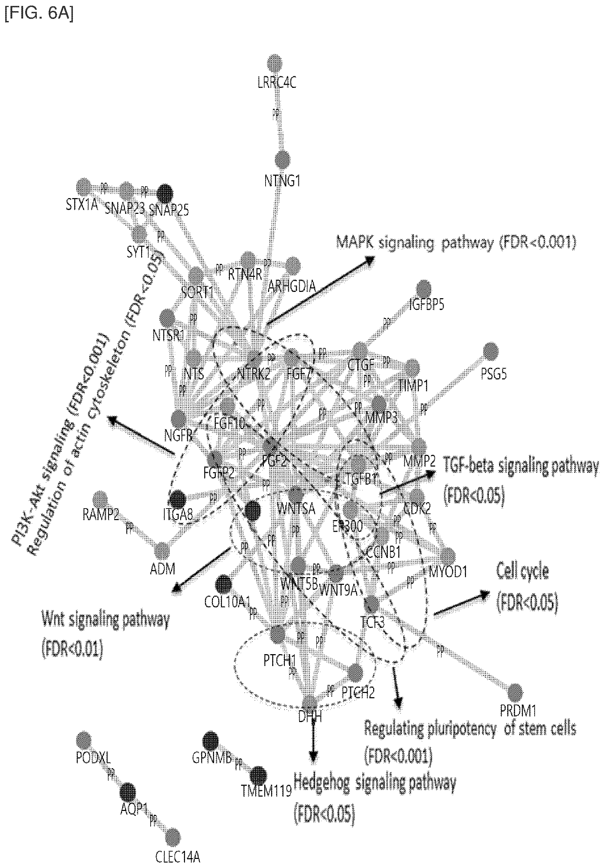

For 3 lots of samples of adipose-derived stem cells (ASC-1. ASC-2. ASC-3) and 3 lots of samples of HB-hair follicle-derived stem cells (HB-HFSC-1, HB-HFSC-2, HB-HFSC-3), a paired-end RNA library was constructed using the TruSeq Stranded mRNA Library Prep Kit according to the manufacturer's protocol, and sequencing was performed at Macrogen (Seoul, Korea). After the pretreatment process, the genes were mapped to the reference genome (version GRCh38) with the HISAT2 program (version 2.1.0, https://ccb.jhu.edu/software/hisat2/index.shtml), and the expression profile for each transcriptome was acquired using the StringTie program (version 2.1.3b, https://ccb.jhu.edu/software/stringtie/) (FIG. 6A). Gene expression levels were handled by taking the logarithm of TPM (Transcripts Per Kilobase Million) values (FIG. 6A). Differentially expressed genes between the control and comparison groups were acquired using the edgeR package [1] of the R program (version 4.0) (1. SZKLARCZYK, Damian, et al. STRING v11: protein-protein association networks with increased coverage, supporting functional discovery in genome-wide experimental datasets. Nucleic acids research, 2019, 47.D1: D607-D613.). In order to compare the expression patterns of genes differentially expressed in stem cells with related disease transcriptome data, publicly available transcriptome data were collected from the GEO (Gene Expression Omnibus) database. Differential gene expression level analysis of each publicly available data was performed using the limma package [2] of the R program (version 4.0) (2. ROBINSON, Mark D.; MCCARTHY, Davis J.; SMYTH, Gordon K. edgeR: a Bioconductor package for differential expression analysis of digital gene expression data. bioinformatics, 2010, 26.1: 139-140.). The statistical test results provided by the analysis program were used as is, and a significance level of less than 0.05 was considered significant. In a case of indicating results with a P value exceeding 0.05, the P value was indicated separately.

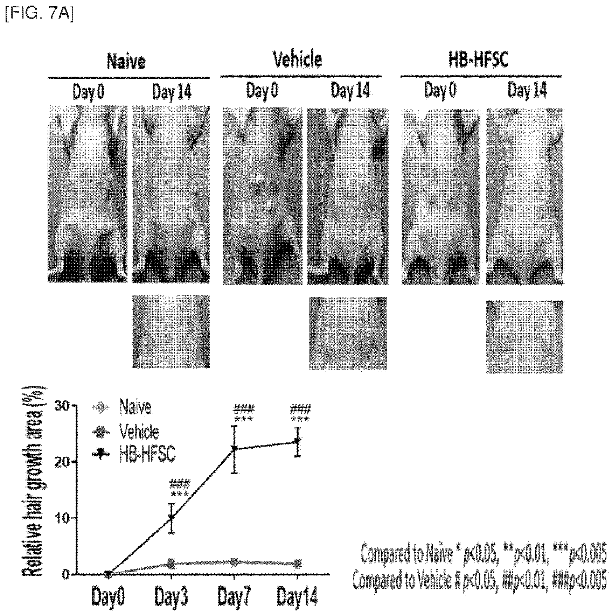

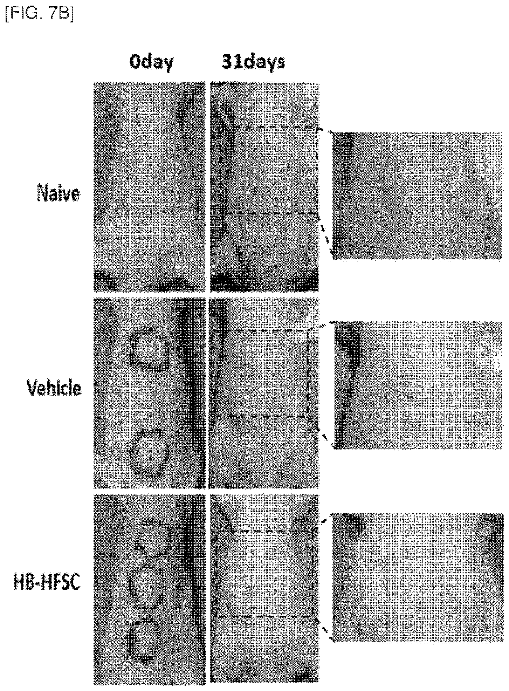

To construct a biological network in which marker genes participate, a protein-protein interaction (PPI) network was constructed using String Database (version 11.5) [3] (3. SMYTH, Gordon K., et al. LIMMA: linear models for microarray data. In Bioinformatics and Computational Biology Solutions Using R and Bioconductor. Statistics for Biology and Health. 2005.) (FIGS. 7A and 7B). The organism reference used to construct the PPI network was Homo sapiens. As settings for constructing a network, the edges connecting nodes representing proteins to each other were connected based on evidence such as experimental results, biological databases and literature, and gene fusion, and an edge score of 0.4 or more was considered significant. After construction of the network, orphan nodes that had no connectivity between other nodes were removed. Only statistically significant biological functions to which node groups belong were extracted based on the KEGG database [4] (4. KANEHISA, Minoru; GOTO, Susumu. KEGG: kyoto encyclopedia of genes and genomes. Nucleic acids research, 2000, 28.1: 27-30.) (False Discovery Rate < 0.05). The test of significant difference between two groups was obtained by performing a T-Test, and a P value < 0.05 was considered significant. The statistical test results provided by the analysis program were used as is, and a significance level of less than 0.05 was considered significant. In a case of indicating results with a P value exceeding 0.05, the P value was indicated separately.

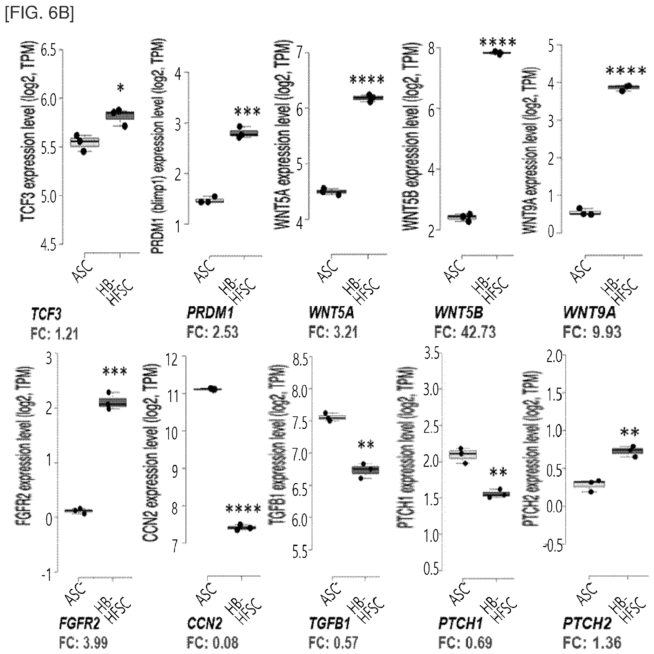

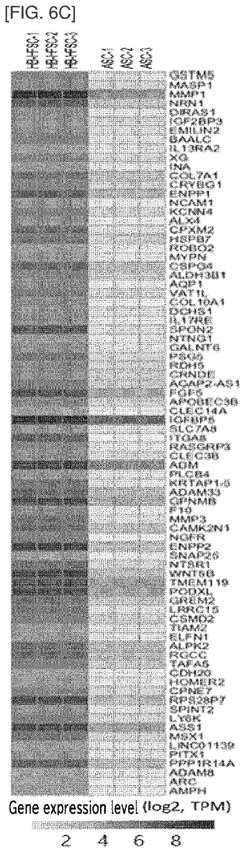

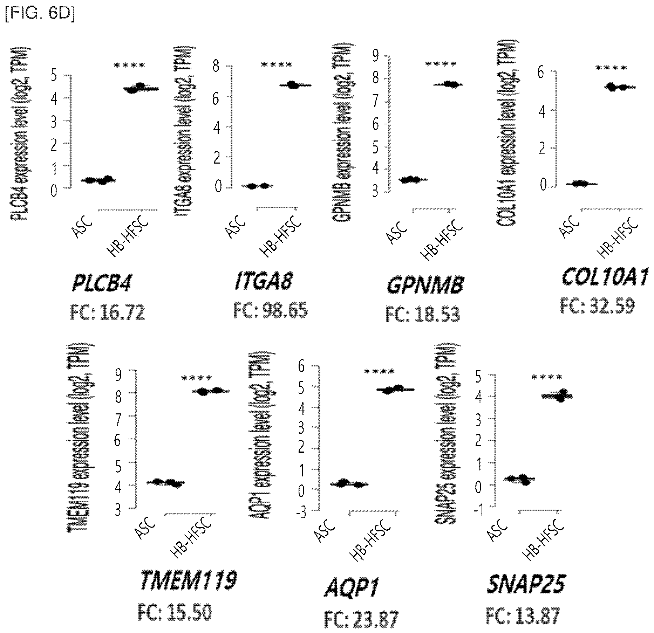

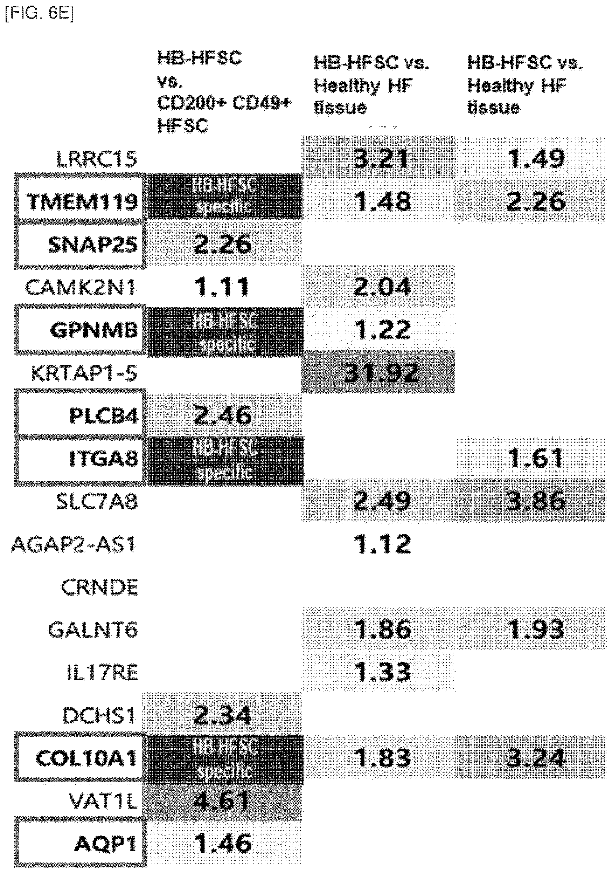

Using String DB, genes expressed in HB-hair follicle-derived stem cells were analyzed with the hair growth promotion-related gene network to examine the correlation with known hair growth promotion pathways, and seven hair growth promotion functional genes (SNAP25, ITGA8, PLCB4, COL10A1, GPNMB, TMEM119, and AQP1) were derived. (FIG. 6A). It was found that HB-hair follicle-derived stem cells expressed major hair growth function-related genes (TCF3, PRDM1, WNT5A, WNT5B, WNT9A, FGFR2, and the like) at higher levels compared to those in adipose-derived stem cells (FIG. 6B). In the HB-hair follicle-derived stem cells, 80 genes expressed with fold change > 10 compared to those in adipose-derived stem cells were identified, and among these, seven hair growth promotion functional genes (SNAP25, ITGA8, PLCB4, COL10A1, GPNMB, TMEM119, and AQP1) derived in FIG. 6A were found to be differentially expressed compared to those in adipose-derived stem cells (FIGS. 6C and 6D). When the gene expression levels of existing hair follicles or hair follicle-derived stem cells were compared with those of HB-hair follicle-derived stem cells, it was found that the expression of seven hair growth promotion functional genes (SNAP25, ITGA8, PLCB4, COL10A1, GPNMB, TMEM119, and AQP1) increased by 10-fold or more and the seven genes were significantly differentially expressed (FIG. 6E).

The seven hair growth promotion functional genes are known to be genes associated with Wnt signaling and PI3K signaling, which are known signalings related to hair follicle formation and growth, and based on this, the hair growth promotion effect of hair follicle-derived stem cells prepared by the hair follicle-derived stem cell preparing method of the present invention can be inferred.

In addition, it was found that the expression of CCN2 and TGFB1 genes, known as genes that induced the catagen phase of hair follicles, decreased (FIG. 6B).

### Example 7. Examination of hair growth promotion effect in nude mice

The experimental animals, 7- to 12-week-old BALB/c nude mice (Central Laboratory Animals), were anesthetized by inhalation with 3% isoflurane (Hana Pharmaceuticals). After it was confirmed that anesthesia was complete, 1 × 10⁶ HB-hair follicle-derived stem cells were suspended in 100 µL of normal saline (NS), and intradermal injection was performed using an insulin syringe (30 gauge) at four corners (2 × 2 cm) at the dorsal part. After administration of HB-hair follicle-derived stem cells, follow-up observation was conducted for 14 and 31 days, and the administration site was photographed using a digital camera immediately after administration (day 0) and on days 3, 7, 14, 28, and 31 after administration. The area of the portion where hair grew was measured based on the percentage of the administration site (2 × 2 cm, 4 cm²) using Image J (NIH). (FIGS. 7A and 7B). From the results, the hair growth promotion effect of the HB-hair follicle-derived stem cells was verified.

### Example 8. Examination of hair follicle formation ability and increase in skin thickness in nude mice

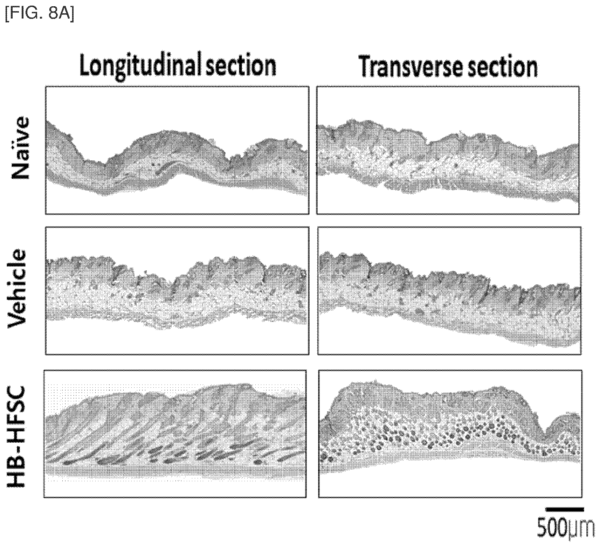

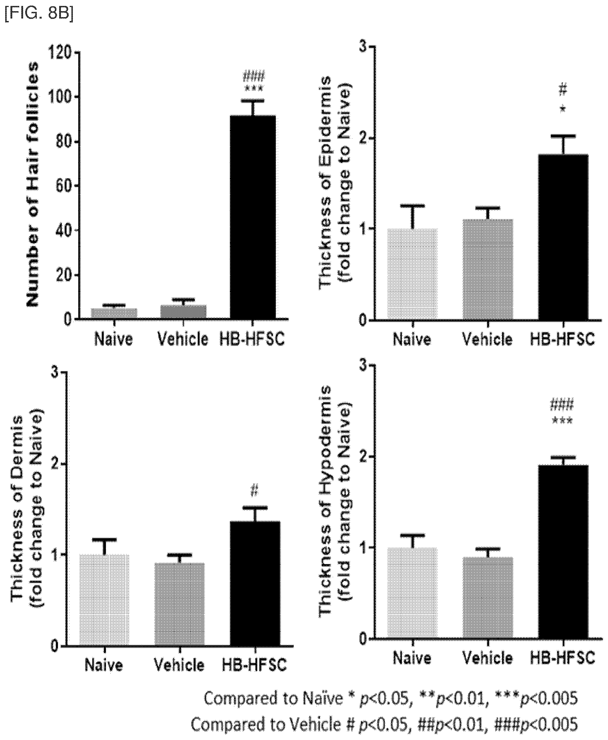

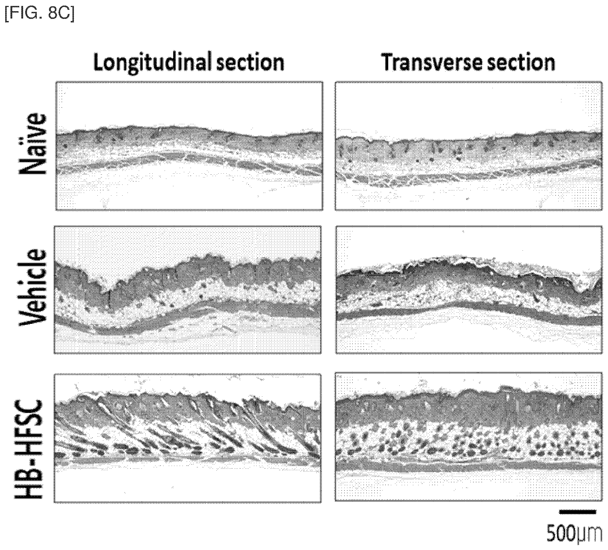

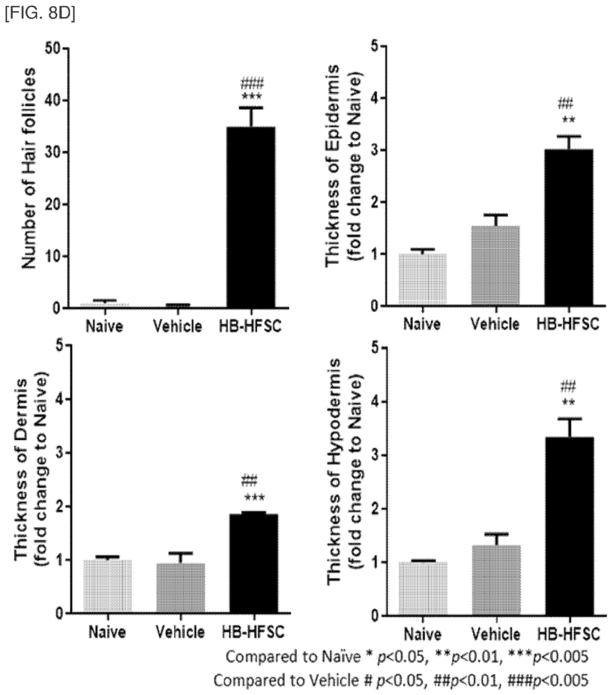

For histological analysis, skin tissue was fixed in a fixative (4% formaldehyde, Sigma, USA) for 24 hours, then the tissue was paraffin embedded and sectioned, and H&E (Hematoxylin & Eosin) staining was performed. Morphological changes in hair follicles and skin layer thickness were examined under an optical microscope. The number of hair follicles formed and the thicknesses of the epidermis, dermis, and hypodermis were measured using Image J (FIG. 8). After 14 and 31 days from the administration of HB-hair follicle-derived stem cells, hair follicle formation and increased skin thickness were verified.

### Example 9. Examination of Wnt signaling mechanism in nude mice

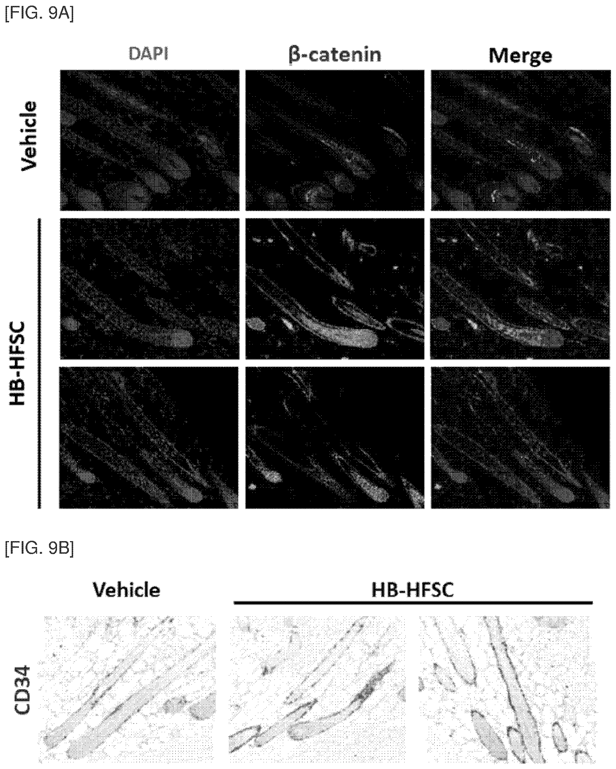

In the same manner as for the H&E staining, the tissues of each group were fixed, paraffin embedded, and sectioned to prepare tissue slides. Immunofluorescence (IF) staining was performed by attaching a secondary antibody to the beta-catenin antibody (Invitrogen, MA 1-2001) (FIG. 9A), the reaction was conducted in a prehybridization solution (50% formamide, 4X SSC, 50 mM DDT, 4X Denhart's soln, 'X TED, 100 ug/ml denatured salmon sperm DNA, 250 ug/ml yeast RNA) at 42 degrees for 1 hour to bind the tissue to CD34 mRNA (FIG. 9B). It was found that β-catenin expression increased and CD34 gene expression increased 31 days after administration of HB-hair follicle-derived stem cells.

Based on the above description, it will be understood by those skilled in the art to which the present invention pertains that the present invention may be implemented in other specific forms without changing the technical spirit or essential features thereof. Therefore, it should be understood that the embodiments described above are not restrictive but illustrative in all aspects. The scope of the present invention is defined by the appended claims rather than by the description preceding them, and therefore all changes and modifications that fall within the metes and bounds of the claims or equivalents of such metes and bounds are intended to be embraced by the claims of the present invention.

## Claims

1. A method for culturing hair follicle-derived stem cells, the method comprising:
(a) encapsulating a hair follicle tissue in a hydrogel and culturing the hair follicle tissue to obtain a culture; and
(b) decomposing the hydrogel in the obtained culture to recover stem cells that have migrated from the hair follicle tissue into the hydrogel and proliferated in the hydrogel.

2. The method for culturing hair follicle-derived stem cells according to claim 1, wherein step (a) involves encapsulating a hair follicle tissue in a double-layered hydrogel and culturing the hair follicle tissue.

3. The method for culturing hair follicle-derived stem cells according to claim 1, wherein a fragment obtained by removing a site corresponding to follicular infundibulum from epidermis is used as the hair follicle tissue.

4. The method for culturing hair follicle-derived stem cells according to claim 1, wherein the hair follicle tissue is cultured for 3 to 20 days in step (a).

5. The method for culturing hair follicle-derived stem cells according to claim 1, wherein a hair follicle tissue is seeded with an inter-tissue spacing of 1 mm or more and 50 mm or less for hair follicle tissue culture in step (a).

6. The method for culturing hair follicle-derived stem cells according to claim 1, wherein the hair follicle-derived stem cells have increased expression of one or more genes selected from SANP25, COL10A1, TMEM119, AQP1, PLCB4, or ITGA8.

7. The method for culturing hair follicle-derived stem cells according to claim 1, wherein the hair follicle-derived stem cells have immunological characteristics in which CD73, CD90, CD105, Nestin, and Lgr5 are expressed but CD34, CD45, and HLA-DR are not expressed.

8. Hair follicle-derived stem cells prepared by the method according to claim 1.

9. The hair follicle-derived stem cells according to claim 8, which have increased expression of one or more genes selected from SANP25, COL10A1, TMEM119, AQP1, PLCB4, or ITGA8.

10. The hair follicle-derived stem cells according to claim 8, which have immunological characteristics in which CD73, CD90, CD105, Nestin, and Lgr5 are expressed but CD34, CD45, and HLA-DR are not expressed.

11. A pharmaceutical composition for hair loss treatment or hair growth promotion, comprising hair follicle-derived stem cells prepared by the method according to claim 1, a culture of the hair follicle-derived stem cells, or cells differentiated from the hair follicle-derived stem cells as an active ingredient.

12. A quasi-drug composition for hair loss improvement or hair growth promotion, comprising hair follicle-derived stem cells prepared by the method according to claim 1, a culture of the hair follicle-derived stem cells, or cells differentiated from the hair follicle-derived stem cells as an active ingredient.

13. A cosmetic composition for hair loss improvement or hair growth promotion, comprising hair follicle-derived stem cells prepared by the method according to claim 1, a culture of the hair follicle-derived stem cells, or cells differentiated from the hair follicle-derived stem cells as an active ingredient.
